# EUROPEAN PATENT APPLICATION

(11) **EP 2 339 368 A2**
(43) Date of publication of application: **29.06.2011**
(21) Application number: 10194131.8
(22) Date of filing: 08.12.2010
(51) Int. Cl.: G01S 7/52, G01S 15/89

(54) **Providing multiple 3-dimensional ultrasound images in an ultrasound image**

(30) Priority: 15.12.2009 KR 20090124957
(71) Applicant: Medison Co., Ltd., Gangwon-do 250-875 (KR)
(72) Inventor: Lee, Suk Jin, 135-851 Seoul (KR); Kim, Jung, 135-851 Seoul (KR)
(74) Representative: Schmid, Wolfgang

(57) **Abstract**

Embodiments for providing a plurality of 3-dimensional ultrasound images in an ultrasound system are disclosed. The ultrasound system includes: an ultrasound data acquisition unit configured to acquire a first ultrasound frame data set from a target object; a processing unit configured to form a 2-dimensional ultrasound image based on the first ultrasound frame data set; and a user input unit for allowing a user to input user input information, wherein the processing unit is further configured to set a plurality of regions of interest (ROIS) in either a multiple ROI setting way or a single ROI setting way based on the user input information, wherein the ultrasound data acquisition unit is further configured to acquire a plurality of second ultrasound frame data sets according to the setting of ROIS, and wherein the processing unit is further configured to form multiple 3-dimensional ultrasound images corresponding to the respective ROIS.

## Description

### CROSS-REFERENCE TO RELATED APPLICATIONS

The present application claims priority from Korean Patent Application No. 10-2009-0124957 filed on December 15, 2009.

### TECHNICAL FIELD

The present disclosure generally relates to ultrasound signal processing, and more particularly to an ultrasound system configured to provide multiple 3-dimensional ultrasound images and a method of implementing the same.

### BACKGROUND

An ultrasound system has been extensively used in the medical field due to its non-invasive and non-destructive nature. Modem high-performance ultrasound imaging diagnostic systems and techniques are commonly used to produce two- or three-dimensional ultrasound images of internal features of patients.

Recently, the ultrasound system may provide a 3-dimensional ultrasound image showing clinical information such as spatial information, anatomical features and the like, which may not be provided by a 2-dimensional ultrasound image. The ultrasound system is designed to transmit ultrasound signals to the target object and then receive ultrasound echo signals. The ultrasound system forms a 2-dimensional ultrasound image based on the received ultrasound echo signals. In response to a user instruction, the ultrasound system sets a region of interest (ROI) on the 2-dimensional ultrasound image. Thereafter, the ultrasound system transmits ultrasound signals to the target object and then receives ultrasound echo signals to thereby form a volume data set corresponding to the ROI. The ultrasound system performs rendering upon the volume data set to form a 3-dimensional ultrasound image of the target object.

Conventionally, after setting the ROI on the 2-dimensional ultrasound image, 3D mode switching should be carried out to form the 3-dimensional ultrasound image corresponding to the ROI. In such a case, when the operator desires to observe another portion in the target object, the 3D mode should be switched to a 2D mode. Thereafter, ROI is set on a 2-dimensional ultrasound image in the 2D mode and the image mode should be again switched to the 3D mode. This, of course, can be very inconvenient and user-unfriendly to the operator.

### SUMMARY

Embodiments for providing a plurality of 3-dimensional ultrasound images in an ultrasound system are disclosed herein. In one embodiment, by way of non-limiting example, an ultrasound system includes: an ultrasound data acquisition unit configured to acquire a first ultrasound frame data set from a target object; a processing unit configured to form a 2-dimensional ultrasound image based on the first ultrasound frame data set; and a user input unit for allowing a user to input user input information, wherein the processing unit is configured to set a plurality of regions of interest (ROIS) in either a multiple ROI setting way or a single ROI setting way based on the user input information, wherein the ultrasound data acquisition unit is configured to acquire a plurality of second ultrasound frame data sets according to the setting of ROIS, and wherein the processing unit is further configured to form multiple 3-dimensional ultrasound images corresponding to the respective ROIS.

In another embodiment, a method of providing a plurality of 3-dimensional ultrasound images in an ultrasound system, comprises: a) acquiring a first ultrasound frame data set from a target object; b) forming a 2-dimensional ultrasound image based on the first ultrasound frame data set; c) setting a plurality of regions of interest (ROIS) in either a multiple ROI setting way or a single ROI setting way based on user input information and forming a plurality of volume data sets corresponding to the respective ROIS; and d) performing rendering upon the respective volume data sets to thereby form a plurality of 3-dimensional ultrasound images.

In yet another embodiment, a computer-readable storage medium storing instructions that, when executed by a computer, cause the computer to provide a method of providing a plurality of 3-dimensional ultrasound images, wherein the method comprises: a) forming a 2-dimensional ultrasound image based on first ultrasound frame data set obtained from a target object; b) setting a plurality of regions of interest (ROIS) in either a multiple ROI setting way or a single ROI setting way based on user input information and forming a plurality of volume data sets corresponding to the respective ROIS; and c) performing rendering upon the respective volume data sets to thereby form a plurality of 3-dimensional ultrasound images.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit.
FIG. 3 is a schematic diagram showing a scanning direction to acquire frame data sets.
FIG. 4 is a schematic diagram showing an example of a multiple ROI setting way.
FIG. 5 is a schematic diagram showing an example of a single ROI setting way.
FIG. 6 is a block diagram showing an illustrative embodiment of a processing unit.
FIG. 7 is a flowchart showing an illustrative embodiment of a procedure of providing a plurality of 3-dimensional ultrasound images.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

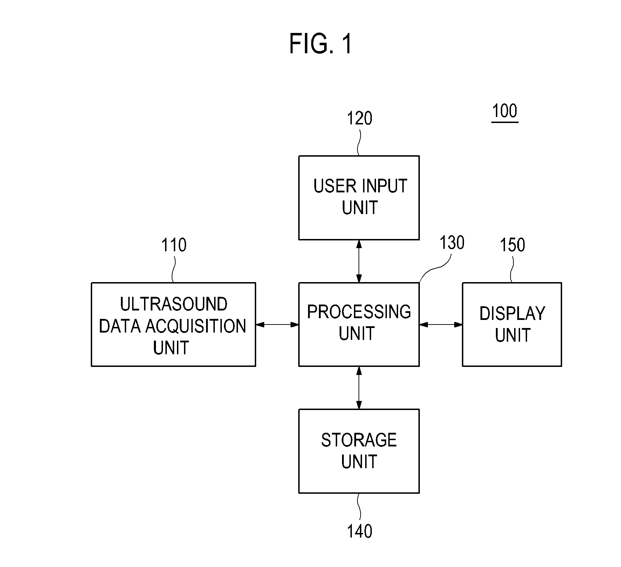

Referring to FIG. 1, an illustrative embodiment of an ultrasound system 100 is shown. As depicted therein, the ultrasound system 100 includes an ultrasound data acquisition unit 110, a user input unit 120, a processing unit 130, a storage unit 140 and a display unit 150. The ultrasound data acquisition unit 110 is configured to transmit ultrasound signals to a target object and receive echo signals reflected from the target object to thereby acquire ultrasound data.

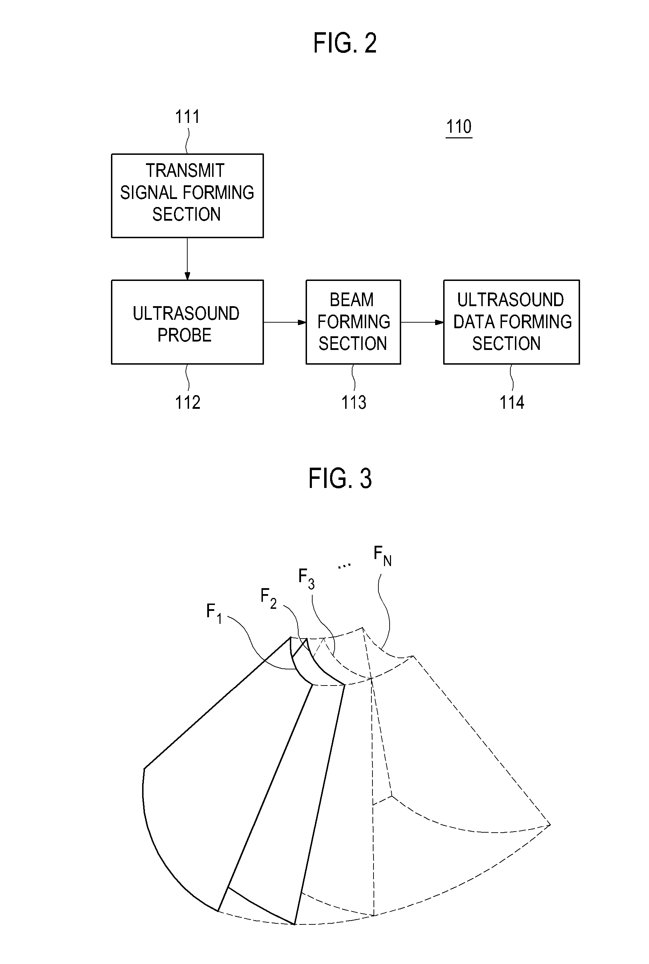

As shown in FIG. 2, the ultrasound data acquisition unit 110 includes a transmit signal forming section 111. The transmit signal forming section 111 is configured to form transmit signals according to image modes such as a brightness mode (B-mode), a Doppler mode (D-mode), a color Doppler mode (C-mode), an elasticity mode, a 3-dimensional mode and the like. The transmit signal forming section 111 repeatedly forms transmit signals in a sequential manner to thereby form a plurality of transmit signals.

The ultrasound data acquisition unit 110 further includes an ultrasound probe 112, which is coupled to the transmit signal forming section 111. The ultrasound probe 112 includes transducer elements configured to output ultrasound signals, which is propagated into the target object, in response to the transmit signals. The ultrasound probe 112 receives echo signals reflected from the target object to thereby output receive signals. The ultrasound probe 112 includes a 3-dimensional mechanical probe, a 2-dimensional probe and the like.

The transmission of the ultrasound signals is controlled by the transmit signal forming section 111. The transmit signal forming section 111 is configured to apply delays to the transmit signals by considering distances between the transducer elements and the focal points.

The ultrasound data acquisition unit 110 includes a beam forming section 113, which is coupled to the ultrasound probe 112. The beam forming section 113 is configured to digitize the receive signals to output digital signals. The beam forming section 113 is further configured to apply delays to the digital signals in consideration of distances between the transducer elements and focal points, thereby forming receive-focused signals. In one embodiment, the receive-focused signals includes first receive-focused signals to obtain a frame data set for forming a 2-dimensional ultrasound image and second receive-focused signals to obtain a plurality of frame data sets for forming a 3-dimensional ultrasound image.

The ultrasound data acquisition unit 110 further includes an ultrasound data forming section 114, which is coupled to the beam forming section 113. The ultrasound data forming section 114 is configured to form a plurality of ultrasound frame data sets based on the receive-focused signals. In one embodiment, the ultrasound frame data sets includes a first ultrasound frame data set corresponding to a 2-dimensional ultrasound image and second ultrasound frame data sets corresponding to a plurality of 2-dimensional ultrasound images Pᵢ(1≤i≤N) for forming a 3-dimensional ultrasound image, as shown in FIG. 3.

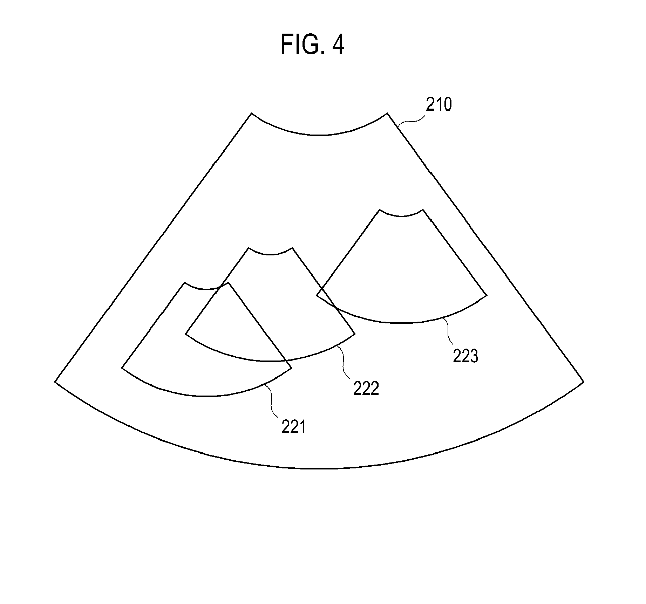

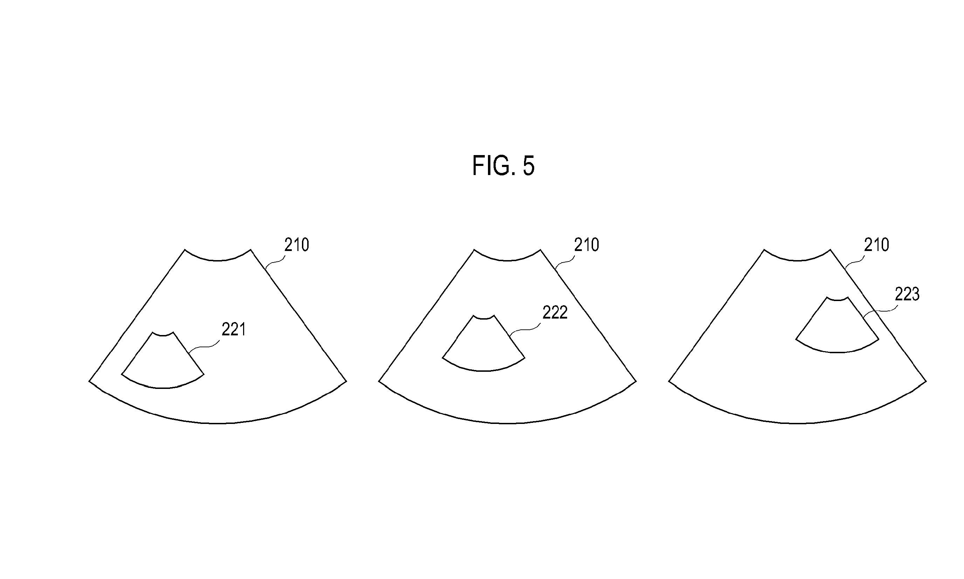

The user input unit 120 is configured to receive user input information. In one embodiment, the user input information includes first input information for selecting either a way of setting a multiple regions of interest (hereinafter, referred to as "multiple ROI setting way") or a way of setting a single ROI (hereinafter, referred to as "single ROI setting way"). The multiple ROI setting way is directed to setting multiple ROIS on a 2-dimensional ultrasound image at the same time. That is, at least one new ROI is set on the 2-dimensional ultrasound image together with a previously set ROI. The single ROI setting way is directed to sequentially setting a single ROI on a 2-dimensional ultrasound image in a way of replacing the ROI on the 2-dimensional ultrasound image with a new ROI. For example, first to third ROIS 221-223 are set on a 2-dimensional ultrasound image 210 at the same time in the multiple ROI setting way, as illustrated in FIG. 4. Further, a first ROI 221, a second ROI 222 and a third ROI 223 are sequentially set on the 2-dimensional ultrasound image in the single ROI setting way, as illustrated in FIG. 5. The 2-dimensional ultrasound image 210 in FIG. 5 is an identical or different ultrasound image.

In one embodiment, the user input information further includes second input information for setting multiple ROIS on the 2-dimensional ultrasound image in the multiple ROI setting way, third input information for requesting an end of the multiple ROI setting way, fourth input information for setting a single ROI on the 2-dimensional ultrasound image in the single ROI setting way, and fifth input information for requesting an end of the single ROI setting way.

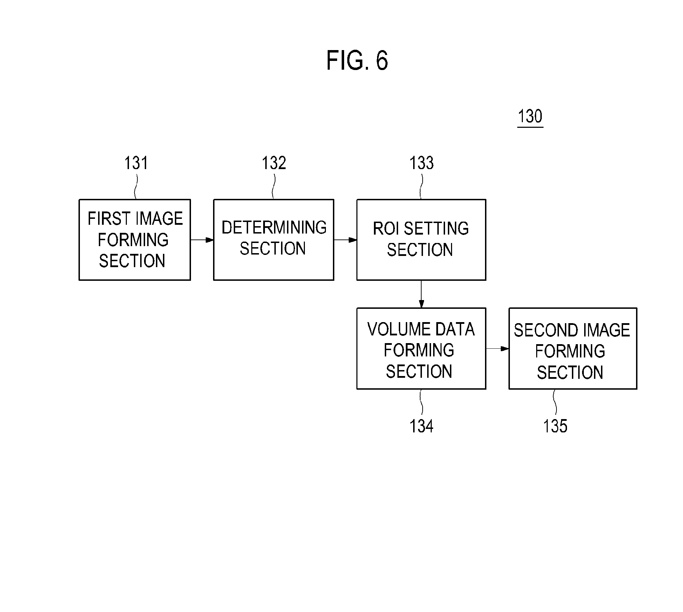

The processing unit 130, which is coupled to the ultrasound data acquisition unit 110 and the user input unit 120, is configured to form a 2-dimensional ultrasound image based on the first ultrasound frame data set. The processing unit 130 is further configured to form a 3-dimensional ultrasound image based on the plurality of frame data sets provided from the ultrasound data acquisition unit 110 according to the user input information. An operation of the processing unit 130 will be described in detail by referring to FIG. 6.

The processing unit 130 includes a first image forming section 131, a determining section 132, a ROI setting section 133, a volume data forming section 134 and a second image forming section 135. The first image forming section 131 is configured to form the 2-dimensional ultrasound image by using the first ultrasound frame data set provided from the ultrasound data acquisition unit 110. In one embodiment, the 2-dimensional ultrasound image is a brightness mode (B-mode) image.

The determining section 132 is configured to analyze the first input information provided from the user input unit 120 to determine a way for setting a plurality of ROIS on the 2-dimensional ultrasound image. In one embodiment, the determining section 132 is configured to output first analysis information for performing the multiple ROI setting way or second analysis information for performing the single ROI setting way.

If the first analysis information is outputted from the determining section 132, then the ROI setting section 133, which is coupled to the determining section 132, sets multiple ROIS on the 2-dimensional ultrasound image based on the second input information in the multiple ROI setting way. On the other hand, if the second analysis information is outputted from the determining section 132, then the ROI setting section 133 sequentially sets a plurality of ROIS on the 2-dimensional ultrasound image based on the fourth input information in the single ROI setting way.

The volume data forming section 134 is configured to form multiple volume data sets corresponding to the respective ROIS by using the plurality of second frame data sets, which are provided from the ultrasound data acquisition unit 110.

The second image forming section 135 is configured to perform rendering upon the respective volume data sets to thereby form multiple 3-dimensional ultrasound images. The rendering includes ray-casting rendering, surface rendering and the like.

Referring back to FIG. 1, the storage unit 140 stores the plurality of volume data sets. Also, the storage unit 140 stores the 3-dimensional ultrasound images. The display unit 150 displays the 2-dimensional ultrasound image formed in the processing unit 130. The display unit 150 further displays the multiple 3-dimensional ultrasound images formed in the processing unit 130. The display unit 150 includes at least one of a cathode ray tube (CRT) display, a liquid crystal display (LCD), an organic light emitting diode (OLED) display and the like.

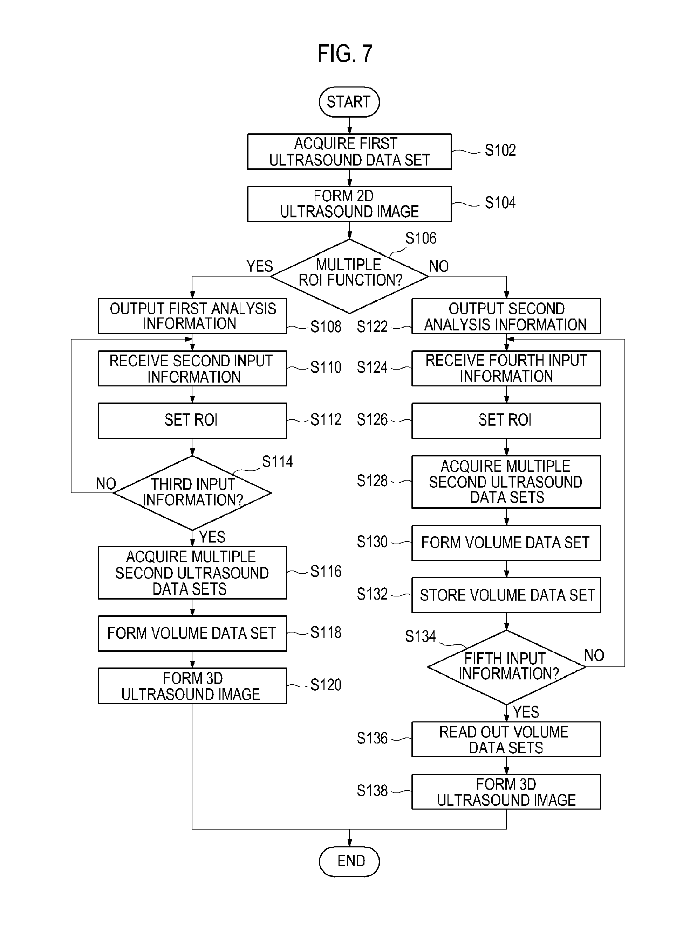

Hereinafter, a process of providing multiple 3-dimensional ultrasound images will be described in detail. FIG. 7 is a flowchart showing an illustrative embodiment of providing multiple 3-dimensional ultrasound images. Referring to FIG. 7, the ultrasound data acquisition unit 110 transmits ultrasound signals to the target object and receives ultrasound echo signals to thereby form a first ultrasound frame data set at step S102. The first ultrasound image forming section 131 forms a 2-dimensional ultrasound image by using the first ultrasound frame data set at step S104.

If the first input information is inputted from the user input unit 120, then the determining section 132 analyzes the first input information to determine which way to set the ROIS is inputted. If it is determined that the multiple ROI setting way is selected at step S106, then the determining section 132 outputs first analysis information to set the ROIS in the multiple ROI setting way at step S108.

If the first analysis information is provided, the ROI setting section 133 receives the second input information from the user input unit 120 at step S110 and then sets multiple ROIS on the 2-dimensional ultrasound image according to the second input information in the multiple ROI setting way at step S112. The ROI setting section 133 checks whether the third input information is inputted through the user input unit 120 at step S114. If it is determined that the third input information is not inputted, then the steps S110 to S112 are repeatedly carried out until the third input information is inputted.

The ultrasound data acquisition unit 110 transmits ultrasound signals to the target object according to the first analysis information and the third input information, and receives ultrasound echo signals reflected from the target object, thereby acquiring multiple frame data sets at step S116. The volume data forming section 134 forms a plurality of volume data sets corresponding to the respective ROIS by using the multiple frame data sets at step S118. The second image forming section 135 performs rendering upon the plurality of volume data sets to form 3-dimensional ultrasound images corresponding to the respective ROIS.

On the other hand, if the second analysis information is outputted from the determining section 132, then the ROI setting section 133 receives the fourth input information from the user input unit 120 at step S124 and set ROIS sequentially on the 2-dimensional ultrasound image based on the fourth input information in the single ROI setting way at step S126.

The ultrasound data acquisition unit 110 transmits ultrasound signals to the target object according to the second analysis information and the fourth input information and receives ultrasound echo signals reflected from the target object, thereby acquiring multiple frame data sets at step S128. The volume data forming section 134 forms a volume data set corresponding to the ROI by using the multiple frame data sets at step S130. The volume data set is stored in the storage unit 140 at step S132.

The ROI setting section 133 checks whether the fifth input information is provided from the user input unit 120 at step S134. The steps S124 to S134 are repeatedly carried out until the fifth input information is inputted through the user input unit 120. If the fifth input information is provided at step S 134, then the second image forming section 135 reads out a plurality of volume data sets from the storage unit 140 at step S136, and performs rendering upon the respective read-out volume data sets, thereby forming a plurality of 3-dimensional ultrasound images at step S138.

Although the above embodiment has been described that the ROI is set by the user input information, the setting of the ROI may not be limited thereto. In another embodiment, the ROI is set on the 2-dimensional ultrasound image according to a present value. In such a case, the present value is stored in the storage unit 140.

In another embodiment, there is a provided a computer-readable storage medium storing instructions that, when executed by a computer, cause the computer to provide a method of providing a plurality of 3-dimensional ultrasound images. The method may comprises a) forming a 2-dimensional ultrasound image based on a first ultrasound frame data set obtained from a target object, b) setting a plurality of regions of interest (ROIS) in either a multiple ROI setting way or a single ROI setting way based on user input information and forming a plurality of volume data sets corresponding to the respective ROIS, and c) performing rendering upon the respective volume data sets to thereby form a plurality of 3-dimensional ultrasound images.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to acquire a first ultrasound frame data set from a target object;
a processing unit configured to form a 2-dimensional ultrasound image based on the first ultrasound frame data set; and
a user input unit for allowing a user to input user input information,
wherein the processing unit is further configured to set a plurality of regions of interest (ROIS) in either a multiple ROI setting way or a single ROI setting way based on the user input information,
wherein the ultrasound data acquisition unit is further configured to acquire a plurality of second ultrasound frame data sets according to the setting of ROIS,
and
wherein the processing unit is further configured to form multiple 3-dimensional ultrasound images corresponding to the respective ROIS.

2. The ultrasound system of Claim 1, wherein the input information includes:
first input information for selecting one of the multiple ROI setting way and
the single ROI setting way;
second input information for setting the ROIS on the 2-dimensional ultrasound image in the multiple ROI setting way;
third input information for requesting an end of the ROI setting of the multiple ROI setting way;
fourth input information for setting the ROIS on the 2-dimensional ultrasound image in the single ROI setting way; and
fifth input information for requesting an end of the ROI setting of the single ROI setting way.

3. The ultrasound system of Claim 2, wherein the processing unit includes:
a first image forming section configured to form the 2-dimensional ultrasound image based on the first ultrasound frame data set;
a determining section configured to analyze the first input information to output analysis information;
a ROI setting section configured to receive the analysis information to set the plurality of ROIS based on the second input information or the fourth input information;
a volume data forming section configured to form volume data sets corresponding to the ROIS by using the plurality of the second ultrasound frame data sets; and
a second image forming section configured to perform rendering upon the respective volume data sets to thereby form the plurality of 3-dimensional ultrasound images.

4. The ultrasound system of Claim 3, wherein the determining section is configured to, when the first input information is input information for selecting the multiple ROI setting way, output first analysis information for performing the multiple ROI setting, and, when the first input information is input information for selecting the single ROI setting way, output second analysis information for performing the single ROI setting.

5. The ultrasound system of Claim 4, wherein the ROI setting section is configured to set the ROI on the 2-dimensional ultrasound image based on the second input information in response to the first analysis information until the third input information is inputted, the ultrasound acquisition unit is configured to transmit ultrasound signals to the target object according to the ROIS set by the second input information to thereby acquire the plurality of second ultrasound frame data sets, and wherein the volume data forming section is configured to form volume data sets corresponding to the respective ROIS by using the plurality of second ultrasound frame data sets.

6. The ultrasound system of Claim 4, wherein the ROI setting section is configured to set the ROI on the 2-dimensional ultrasound image based on the fourth input information in response to the second analysis information, the ultrasound acquisition unit is configured to transmit ultrasound signals to the target object according to the ROIS set by the fourth input information to thereby acquire the plurality of second ultrasound frame data sets, and the volume data forming section is configured to form volume data sets corresponding to the respective ROIS by using the plurality of second ultrasound frame data sets, and wherein the ROI setting section, the ultrasound data acquisition unit and the volume data forming section are configured to repeatedly perform the ROI setting, the second ultrasound data acquisition and the volume data formation until the fifth input information is inputted.

7. The ultrasound system of Claim 6, wherein the second image forming section is configured to read out the plurality of volume data sets from a storage unit and perform rendering upon the read-out volume data sets to thereby form the plurality of 3-dimensional ultrasound images.

8. A method of providing a plurality of 3-dimensional ultrasound images in an ultrasound system, comprising:
a) acquiring a first ultrasound frame data set from a target object;
b) forming a 2-dimensional ultrasound image based on the first ultrasound frame data set;
c) setting a plurality of regions of interest (ROIS) in either a multiple ROI setting way or a single ROI setting way based on user input information and forming a plurality of volume data sets corresponding to the respective ROIS; and
d) performing rendering upon the respective volume data sets to thereby form a plurality of 3-dimensional ultrasound images.

9. The method of Claim 8, wherein the input information includes:
first input information for selecting one of the multiple ROI setting way and
the single ROI setting way;
second input information for setting the ROIS on the 2-dimensional ultrasound image in the multiple ROI setting way;
third input information for requesting an end of the ROI setting of the multiple ROI setting way;
fourth input information for setting the ROIS on the 2-dimensional ultrasound image in the single ROI setting way; and
fifth input information for requesting an end of the ROI setting of the single ROI setting way.

10. The method of Claim 9, wherein the step c) includes:
c1) receiving the first input information from a user;
c2) analyzing the first input information to output analysis information; and
c3) forming the volume data sets corresponding to the respective ROIS based on the second input information or the fourth input information in response to the analysis information.

11. The method of Claim 10, wherein the step c2) includes:
outputting, when the first input information is input information for selecting the multiple ROI setting way, first analysis information for performing the multiple ROI setting; and
outputting, when the first input information is input information for selecting the single ROI setting way, second analysis information for performing the single ROI setting.

12. The method of Claim 11, wherein the step c3) includes:
c31) receiving the second input information in response to the first analysis information;
c32) setting a plurality of ROIS on the 2-dimensional ultrasound image based on the second input information;
c33) repeatedly performing the steps c31) and c32) until the third input information is inputted;
c34) acquiring, when the third input information is inputted, the plurality of second ultrasound frame data sets from the target object; and
c35) forming the volume data sets corresponding to the respective ROIS by using the plurality of second ultrasound frame data sets.

13. The method of Claim 11, wherein the step c3) includes:
c31) receiving the fourth input information in response to the second analysis information;
c32) setting the ROI on the 2-dimensional ultrasound image based on the fourth input information;
c33) acquiring the plurality of second ultrasound frame data sets;
c34) forming volume data sets corresponding to the respective ROIS by using the plurality of second ultrasound frame data sets; and
c35) repeatedly perform the steps c31) to c34) until the fifth input information is inputted.

14. The method of Claim 13, wherein the step d) includes:
reading out the plurality of volume data sets from a storage unit; and
performing rendering upon the read-out volume data sets to thereby form the plurality of 3-dimensional ultrasound images.

15. A computer-readable storage medium storing instructions that, when executed by a computer, cause the computer to provide a method of providing a plurality of 3-dimensional ultrasound images, the method comprising:
a) forming a 2-dimensional ultrasound image based on a first ultrasound frame data set obtained from a target object;
b) setting a plurality of regions of interest (ROIS) in either a multiple ROI setting way or a single ROI setting way based on user input information and forming a plurality of volume data sets corresponding to the respective ROIS; and
c) performing rendering upon the respective volume data sets to thereby form a plurality of 3-dimensional ultrasound images.
